# EUROPÄISCHE PATENTSCHRIFT

(11) **EP 4 051 218 B1**
(45) Veröffentlichungstag und Bekanntmachung des Hinweises auf die Patenterteilung: **25.06.2025**
(21) Anmeldenummer: 20790275.0
(22) Anmeldetag: 13.10.2020
(51) Int. Cl.: A61K 8/19, A61K 8/20, A61K 8/34, A61K 8/36, A61K 8/39, A61K 8/86, A61Q 15/00, A61K 8/06

(54) **KOSMETISCHE ZUBEREITUNG UMFASSEND ERDALKALIMETALLSALZE, CARBONSÄUREN UND EMULGATOREN MIT VERZWEIGTER HYDROPHOBER KETTE**
COSMETIC PREPARATION COMPRISING ALKALINE EARTH METAL SALTS, CARBOXYLIC ACIDS AND EMULSIFIERS HAVING BRANCHED HYDROPHOBIC CHAINS
PRÉPARATION COSMÉTIQUE COMPRENANT DES SELS DE MÉTAUX ALCALINO-TERREUX, DES ACIDES CARBOXYLIQUES ET DES ÉMULSIFIANTS À CHAÎNES HYDROPHOBES RAMIFIÉES

(30) Priorität: 28.10.2019 DE 102019216547
(43) Veröffentlichungstag der Anmeldung: 07.09.2022
(73) Patentinhaber: Beiersdorf AG, 22529 Hamburg (DE)
(72) Erfinder: NEUMANN, Lätitia, 21149 Hamburg (DE); KLAUCK, Robert, 21465 Reinbek (DE); BRAUN, Melanie, 20535 Hamburg (DE)
(74) Vertreter: Beiersdorf AG
(86) Internationale Anmeldenummer: PCT/EP2020/078667
(87) Internationale Veröffentlichungsnummer: WO 2021/083657

(56) Entgegenhaltungen:
- WO-A2-2009/012925
- WO-A2-2013/013999
- DE-A1- 102007 063 351
- DATABASE GNPD [online] MINTEL; 20 September 2012 (2012-09-20), ANONYMOUS: "Refreshing Hydrating Set", XP055756769, retrieved from https://www.gnpd.com/sinatra/recordpage/1874944/ Database accession no. 1874944
- DATABASE GNPD [online] MINTEL; 13 January 2015 (2015-01-13), ANONYMOUS: "Shining & Whitening Emulsion", XP055756823, Database accession no. 2907589
- DATABASE GNPD [online] MINTEL; 11 February 2008 (2008-02-11), ANONYMOUS: "Matte But Never Dull Pomade", XP055756774, Database accession no. 859096
- DATABASE GNPD [online] MINTEL; 20 September 2012 (2012-09-20), ANONYMOUS: "Refreshing Hydrating Set", XP055756769, Database accession no. 1874944

## Beschreibung

Die Erfindung ist eine Kombination aus einem oder mehreren Erdalkalimetallsalzen, ein oder mehreren Carbonsäuren und ein oder mehrere Emulgatoren mit verzweigter hydrophober Kette in kosmetischen oder dermatologischen Zubereitungen.

Die erfindungsgemäßen Zubereitungen zeigen einen synergistischen schweißhemmenden Effekt und lassen sich als Spray oder Roll-on applizieren.

Idealerweise liegt der pH-Wert der Zubereitung unter 5.

Im Zuge der Entwicklung antitranspirant wirksamer Zubereitungen ist beobachtet worden, dass Erdalkalimetallsalze zusammen mit Fettsäuren eine antitranspirante Wirkung aufweisen können. Dazu werden idealerweise die Fettsäuren in der Zubereitung in möglichst hohen Mengen eingesetzt.

Nun wurde allerdings auch festgestellt, dass die Fettsäuren eine verdickende Eigenschaft auf die Zubereitungen, insbesondere auf Emulsionen basierende Zubereitungen, ausüben. Für übliche kosmetische Produktformate wie Roll-On und Sprays sind aber möglichst dünnflüssige Systeme erforderlich.

Es war daher eine Aufgabe der vorliegenden Erfindung die Viskosität der Zubereitungen, enthaltend Erdalkalimetallsalze und Fettsäuren abzusenken und gleichzeitig die schweißhemmende Wirkung aufrecht zu erhalten.

In der Literatur wird berichtet, dass Calcium- und Magnesiumsalze keine effektiven Antitranspirant Wirkstoffe seien (Reller, Lüdders, Pharmacologic and Toxicologic Effects of topically applied agents on the eccrine sweat glands, Vol 4, p 18, 1975).

In der WO 2013013999 A2 wird jedoch die antitranspirante Wirksamkeit (Schweißreduktion) von Erdalkalimetallsalzen beschrieben. Als AT- wirksam werden darin Verbindungen aus polyvalente Kationen, wie Beryllium, Magnesium, Calcium, Strontium, Barium, Titanium, Mangan, Zink, Hafnium und Aluminium, mit Anionen aus der Gruppe der Halogenide sowie Carbonsäuren aufgeführt und zwar nur die Salze Acetat, Propionat, Pyrrolidoncarboxylat, Sorbat, Gluconat, Ascorbat, Pantothenat, Citrat, Lactat, Aspartat, Glutamat, Bicarbonat oder Nitrat.

In der DE 102014222270 A1 werden Aluminiumsalzfreie Antitranspirant-Zusammensetzungen beschrieben. Der AT-Effekt soll auf den Einsatz von Hydroxycarbonsäuren oder deren Salze beruhen. Als mögliche Hydroxysäuren werden u.a. Zitronensäure, Äpfelsäure, Gallussäure und Weinsäure offenbart.

In der WO 2009012925 A2 wird eine Deodorantzubereitung enthaltend Wasser (a), einen Antitranspirant-Wirkstoff wie Aluminiumchlorhydrat, Mandelsäure (c), Emulgatoren wie PEG-40 hydrogenated castor oil und Magnesiumchlorid und/oder Calciumchlorid (b) beschrieben.

DE 102005012476 A1 beschreibt die Verwendung von ausgewählten C2-C12 Carbonsäuren und deren Metallsalze als den an der Körpergeruchsbildung beteiligten Keime inhibierende Substanzen. Eine antitranspirante Wirkung wird erst durch den Zusatz üblicher Aluminiumsalze erreicht.

DE 102015226630 A1 offenbart Kieselsäure in einem 2-Kammerpackmittel zur Schweißreduktion. Carbonsäuren, wie Weinsäure Zitronensäure oder Milchsäure werden hierin als Puffersubstanzen beschrieben. Der pH-Wert der Kieselsäurehaltigen Zubereitungen liegt bevorzugt bei 5,1 bis 6,9.

Wünschenswert ist es neue antitranspirant wirkende Zubereitungen zur Verfügung zu stellen, die die Nachteile der bekannten AT-wirksamen Zubereitungen nicht aufweisen. Insbesondere ist es wünschenswert alternative AT- Zubereitungen zur Verfügung zu stellen, die keine Aluminiumsalze umfassen.

Wünschenswert ist es kosmetische oder dermatologische antitranspirant wirksame Zubereitung mit Erdalkalisalzen und Carbonsäure als AT-wirker zur Verfügung zu stellen, die als Spray oder Roll-on topisch applizierbar sind.

Die Erfindung ist eine kosmetische oder dermatologische wasserhaltige und/oder alkoholische Zubereitung umfassend ein oder mehrere Erdalkalimetallsalze, ein oder mehrere Carbonsäuren und ein oder mehre Emulgatoren mit verzweigter hydrophober Kette.

Als Emulgatoren mit verzweigter hydrophober Kette werden ein oder mehrere Emulgatoren gewählt aus der Gruppe der Gemische aus Polyethylenglycolethern mit verzweigten Stearylalkoholen, insbesondere mit der Struktur (I) 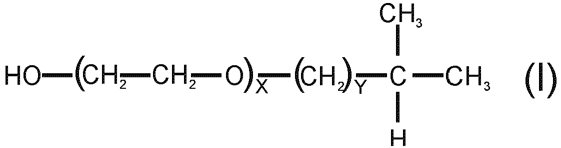 wobei x im Bereich von 15 bis 25, insbesondere 20, und y im Bereich 10 bis 20, insbesondere15 zu wählen sind.

Bevorzugt wird als erfindungsgemäßer Emulgator Isosteareth-20 (Polyoxyethylene (20) isostearyl ether (CAS 52292-17-8)) gewählt.

Durch Zusatz von mindestens einem Emulgator mit verzweigter hydrophober Kette, aus der Gruppe der Gemische aus Polyethylenglycolethern mit verzweigten Stearylalkoholen, insbesondere der Struktur (I), insbesondere Isosteareth-20, zu einem ansonsten beispielsweise linearen Emulgatorsystem, konnte die Viskosität der sie enthaltenen Zubereitungen deutlich gesenkt werden, so dass es möglich ist, bei genügend hohen Gehalten an wirksamen Fettsäuren noch eine Anwendung in Roll-On und Sprays zu ermöglichen. Bei den verzweigten PEG-Emulgatoren funktioniert besonders gut Isosteareth-20.

Erstaunlich war, dass andere Emulgatoren, die zwar ähnlich aufgebaut sind wie die zu der Problemlösung beitragenden Emulgatoren, wie beispielsweise Isosteareth-20, das Problem der Viskositätsabsenkung aber nicht lösen, da sie keinen oder nur einen geringen Einfluss auf die Viskosität genommen haben.

Dazu wurden folgende Viskositätsuntersuchungen mit erfindungsgemäßen Zubereitungen und nicht-erfindungsgemäßen Zubereitungen durchgeführt.

| | Formel 1 | Formel 2 | Formel 3 | Formel 4 | Formel 5 |
|---|---|---|---|---|---|
| Persea Gratissima Oil | 0,1 | 0,1 | 0,1 | 0,1 | 0,1 |
| Steareth-21 | 1,4 | 1,2 | 1 | 2,2 | 2 |
| Steareth-2 | 4,1 | 4,1 | 4,5 | 3,3 | 3,5 |
| Octyldodecanol | 0,1 | 0,1 | 0,1 | 0,1 | 0,1 |
| PPG-15 Stearyl Ether | 3 | 3 | 3 | 3 | 3 |
| Phenoxyethanol | 0,3 | 0,3 | 0,3 | 0,3 | 0,3 |
| Isosteareth-20 | 1 | | | | |
| PEG-100 Stearate | | | 1 | | |
| PEG-40 Stearate | | 1 | | | |
| Ceteareth-12 | | | | | 1 |
| Ceteareth-3 | | | | 1 | |
| Palmitin acid+Stearic acid+Myristic acid arachidic acid+oleis acid | 2 | 2 | 2 | 2 | 2 |
| Magnesium Chlorid | 5 | 5 | 5 | 5 | 5 |
| Propylene Glycol | 3 | 3 | 3 | 3 | 3 |
| Octenidine HCl | 0,5 | 0,5 | 0,5 | 0,5 | 0,5 |
| Parfuem | 1 | 1 | 1 | 1 | 1 |
| Aqua | add 100 | add 100 | add 100 | add 100 | add 100 |
| Citric acid | pH=4 | pH=4 | pH=4 | pH=4 | pH=4 |
| Viskosität nach Herstellung | 150mPas | 400mPas | 400mPas | 500mPas | 450mPas |
| Viskosität nach 7d | 1700mPas | 2950mPas | 3000mPas | 3500mPas | 32500mPas |

| | Formel 1 | Formel 6 | Formel 7 | Formel 8 | Formel 9 |
|---|---|---|---|---|---|
| Persea Gratissima Oil | 0,1 | 0,1 | 0,1 | 0,1 | 0,1 |
| Steareth-21 | 1,4 | 2 | 2 | 1,4 | 2 |
| Steareth-2 | 4,1 | 3,5 | 3,5 | 4,1 | 3,5 |
| Octyldodecanol | 0,1 | 0,1 | 0,1 | 0,1 | 0,1 |
| PPG-15 Stearyl Ether | 3 | 3 | 3 | 3 | 3 |
| Phenoxyethanol | 0,3 | 0,3 | 0,3 | 0,3 | 0,3 |
| Isosteareth-20 | 1 | | | | |
| Laureth-4 | | | | | 1 |
| PEG-7 Hydrogenated Castor Oil | | | 1 | | |
| PEG-40 Hydrogenated Castor Oil | | | | 1 | |
| Isoceteth-20 | | 1 | | | |
| Palmitin acid+Stearic acid+Myristic acid arachidic acid+oleis acid | 2 | 2 | 2 | 2 | 2 |
| Magnesium Chlorid | 5 | 5 | 5 | 5 | 5 |
| Propylene Glycol | 3 | 3 | 3 | 3 | 3 |
| Octenidine HCl | 0,5 | 0,5 | 0,5 | 0,5 | 0,5 |
| Parfuem | 1 | 1 | 1 | 1 | 1 |
| Aqua | add 100 | add 100 | add 100 | add 100 | add 100 |
| Citric acid | pH=4 | pH=4 | pH=4 | pH=4 | pH=4 |
| Viskosität nach Herstellung | 150mPas | 500mPas | 350mPas | 400mPas | 500mPas |
| Viskosität nach 7d | 1700mPa s | 2900mPa s | 3500mPa s | 2850mPa s | 3100mPa s |

Direkt nach der Herstellung aber vor allem nach einer Lagerung von 7 Tagen wird der erfindungsgemäße Effekt und damit der erfindungsgemäße Vorteil und Unterschied deutlich. Der gezeigte Effekt zeigte sich auch in anderen erfindungsgemäßen Zubereitungen mit Calciumsalzen und/oder Carbonsäuren, wie Adipinsäure, Arachinsäure, Bernsteinsäure, oder Laurinsäure.

Erfindungsgemäß ist daher auch die Verwendung von ein oder mehrere Emulgatoren aus der Gruppe der Gemische aus Polyethylenglycolethern mit verzweigten Stearylalkoholen, insbesondere Isosteareth-20, in kosmetischen oder dermatologischen Zubereitungen umfassend Wasser und/oder Alkohol, ein oder mehrere wasserlösliche Erdalkalimetallsalze und ein oder mehrere Carbonsäuren, zur Verringerung der Viskosität der Zubereitung.

Der Anteil an ein oder mehreren Emulgatoren (I), insbesondere Isosteareth-20, wird vorteilhaft im Bereich von 0,5 bis 2 Gew.%, bezogen auf die Gesamtmasse der Zubereitung, gewählt.

Die der Zubereitung zugesetzten Erdalkalimetallsalze sind wasserlöslich.

Der pH-Wert der Zubereitung wird auf einen Wert von kleiner 5, insbesondere im Bereich von 4,5 und kleiner eingestellt.

Die erfindungsgemäße Zubereitung ist bevorzugt antitranspirant wirksam ohne dass weitere schweißhemmende Substanzen, insbesondere Aluminiumsalze, vorhanden sind.

Die Verwendung der erfindungsgemäßen Zubereitungen zur Schweißhemmung und als Antitranspirantien ist ebenso Teil der vorliegenden Erfindung.

Die erfindungsgemäßen Zubereitungen dienen vorteilhaft zur Schweißreduktion vornehmlich in der Achsel. Daneben können die Formulierungen ebenfalls eingesetzt werden um an anderen Körperarealen, an denen Verbraucher unerwünscht schwitzen, den Schweißfluss zu verringern. Das können, ohne sich darauf zu beschränken, die Füße, die Stirn, die Handinnenflächen oder der Rücken sein. Aufgrund der vorteilhaften Aluminiumfreiheit lassen sich die erfindungsgemäßen Zubereitungen problemlos auch auf diesen zum Teil empfindlichen Hautarealen anwenden.

Appliziert werden die Formulierungen in einer Form, die für den Verbraucher akzeptabel ist, das können vorteilhaft beispielsweise Pumpsprays, Sprays mit Treibgas, Druckluftsprays, Kugelapplikatoren (Roll-on) sein.

Aufgrund der vorteilhaften Viskositätsbeeinflußung ist daher auch die Verwendung einer erfindungsgemäßen Zubereitung in Spray- oder Roll-on Applikatoren erfindungsgemäß.

Eine häufige Zubereitungsform kosmetischer Zubereitungen sind Emulsionen.

Erdalkalimetallsalze wie Magnesiumstearat sind in kosmetischen Zubereitungen bekannt. So kann beispielsweise Magnesiumstearat zur Erhöhung der Konsistenz in Ölphasen von Emulsionen eingesetzt werden. Ein typisches Beispiel ist die bekannte Nivea Creme.

Diese üblichen kosmetischen Formulierungen besitzen einen pH-Wert, der höher als der pKs-Wert von Fettsäuren liegt. Somit bildet sich aus Erdalkalimetallsalzen, wie den Chloriden, und dem Anion der Fettsäure das nur in der Ölphase vorliegt, schwerlösliche Erdalkalimetallsalz der Fettsäure, welches dann stark die Viskosität erhöht.

Es war daher auch das Ziel, dass sich die schwerlöslichen Erdalkalimetallsalze nicht schon in der Zubereitung bilden.

Erfindungsgemäß werden dazu zum Unterschied des Standes der Technik die schwerlöslichen Erdalkalimetallsalze der Carbonsäuren nicht direkt zugesetzt, sondern lösliche Erdalkalimetallsalze, wie Magnesiumchlorid, werden in der Wasserphase und die Carbonsäuren in der Ölphase vorgelegt und der pH-Wert wird vorzugsweise unter 5 eingestellt. In Emulsionen, die eine oder mehrere Wasser bzw. Ölphasen umfassen, liegen damit die wasserlöslichen Erdalkalisalze, wie Magnesiumchlorid, in der Wasserphase und die Carbonsäure, wie beispielsweise Stearinsäure, undissoziiert in der Ölphase vor.

Denn wird der pH-Wert der Formulierungen deutlich über den pKs-Wert der Fettsäure eingestellt bildet sich bereits in der Zubereitung das Salz der Fettsäure und so steigt die Viskosität stark an, was wiederum als starker Verdicker für Ölphasen bekannt ist, was zu vermeiden ist.

Daher wird der pH-Wert der Zubereitung im Bereich von weniger als 5, insbesondere weniger als 4,5 eingestellt, und es wird überraschenderweise keine Ausfällung des schwerlöslichen Magnesium-Fettsäuresalzes beobachtet. In einer Emulsion befinden sich die beiden Komponenten erfindungsgemäß in unterschiedlichen Phasen und können so gemeinsam auf die Haut aufgebracht werden.

Wird die erfindungsgemäße Zubereitung dann auf die Haut aufgetragen, kommt es aufgrund des Haut pH-Bereiches von 5 bis 7 zu einer pH-Verschiebung der auf der Haut aufgetragenen Zubereitung in den Bereich von größer 4 bzw. größer 5. Aufgrund der pH-Verschiebung wird eine Ausfällung der schwerlöslichen Erdalkalimetallfettsäuresalze, wie z.B. Magnesiumstearat, beobachtet.

Durch die Auftragung der erfindungsgemäßen Zubereitung auf die Haut bilden sich Erdalkalipräzipitate, die in den Schweißdrüsenausfuhrgängen ausfallen und so zu einer Verengung oder Verstopfung führen und so zu einer Hemmung der Schweißbildung beitragen. Aufgrund der pH-Wert Erhöhung nach dem Verteilen auf der Haut erfolgt der Schritt der Deprotonierung der Säure, die die Präzipitatbildung bedingt. In der Formulierung befindet sich bspw. Stearinsäure, die, sobald das Produkt auf die Haut aufgetragen wird, dissoziiert und zusammen mit dem Magnesiumchlorid das unlösliche Magnesiumstearat bildet, was dann schließlich für die Schweißreduktion verantwortlich ist.

So zeigte eine bevorzugte Emulsionszubereitung mit Magnesiumchlorid und Stearinsäure und einem pH-Wert von 4,0 eine äußerst gute schweißhemmende Wirkung.

Die erfindungsgemäße Zubereitung weist einen pH-Wert von kleiner 5 auf, bevorzugt zwischen 3,5 und 4,5, insbesondere im Bereich von 3,8 bis 4,2, so dass die Zubereitung ohne Ausfällungen gelagert und anwendungsbereit zur Verfügung steht.

Für die pH-Wert Einstellung von Zubereitungen sind dem Fachmann zahlreiche Möglichkeiten geläufig. Insbesondere der Zusatz der Carbonsäuren ermöglicht und erzeugt schon einen pH-Wert im bevorzugten Bereich.

Der erfindungsgemäß niedrige pH-Wert zeigte sich überraschenderweise auch vorteilhaft in Bezug auf die Konsistenz der Zubereitung.

Erfindungsgemäß wird zwischen den beiden Erdalkalimetallsalzarten unterschieden.

Die in Wasser löslichen Erdalkalimetallsalze, wie die bevorzugten Magnesium-, Calcium- und Strontiumhalogenide, werden der Zubereitung zugesetzt.

Erst nach der Auftragung auf der Haut bilden sich die schwerlöslichen Erdalkalimetallsalze der Fettsäuren, wie beispielsweise Magnesium-, Calcium oder Strontiumstearat, - palmitat,-myristat,- oder oleat. Letztere, die als schwerlösliche Erdalkalimetallsalze bezeichneten Salze, sind nicht als diejenigen Salze zu verstehen, die als lösliche Erdalkalimetallsalze der Zubereitung von vornherein zugesetzt werden. Erst die letztgenannten schwerlöslichen Salze bilden die als Präzipitate bezeichneten Ausfällungen auf der Haut, die für die AT-wirkung verantwortlich sind. Zubereitungen umfassend wasserlösliche Salze alleine zeigen die AT-wirkung und Präzipitatbildung nicht. Zubereitungen umfassend allein die schwerlöslichen Erdalkalisalze wirken wiederum nicht schweißhemmend, da die schwerlöslichen Salze nicht zu einer Präzipatbildung auf der Haut zur Verfügung stehen.

Insbesondere sind die Verfahren bevorzugt, in denen als Erdalkalimetallsalze Magnesium- oder Calciumchloride und als Carbonsäuren ein oder mehrere Säuren gewählt aus der Gruppe Adipinsäure, Arachinsäure, Laurinsäure, Myristinsäure, Caprylsäure, Caprinsäure, Ölsäure, Plamitinsäure, Behensäure, Linolensäure, Stearinsäure und/oder Weinsäure, insbesondere Stearinsäure und Palmitinsäure, gewählt werden.

Als erfindungsgemäß bevorzugte Carbonsäuren werden ein oder mehrere Säuren aus der Gruppe gebildet aus Adipinsäure, Arachinsäure, Bernsteinsäure, Laurinsäure, Maleinsäure, Malonsäure, Myristinsäure, Ölsäure, Palmitinsäure, Stearinsäure und/oder Weinsäure gewählt. Bevorzugt ist eine Mischung aus mehreren dieser Säuren zu wählen, insbesondere eine Mischung umfassend Palmitinsäure, Stearinsäure, Myristinsäure, Ölsäure und Arachinsäure.

Als Erdalkalisalze werden bevorzugt eingesetzt Magnesium-, Calcium- und Strontiumhalogenide, insbesondere Magnesiumchlorid und seine Hydrate, insbesondere Hydrate bis MgCl₂*6H₂O, insbesondere MgCl₂*6H₂O, und/oder Calciumchlorid, sowie Magnesiumphosphat oder Magnesiumlactat.

Die erfindungsgemäßen Zubereitungen unterscheiden sich von Zubereitungen des Standes der Technik, die Erdalkalimetallsalze der zitierten Carbonsäuren umfassen, wie beispielsweise Magnesiumstearat, allein dadurch, dass die beiden erfindungswesentlichen Substanzen, wasserlösliches Erdalkalisalz und Carbonsäure getrennt voneinander vorhanden sind.

Zubereitungen, die Erdalkalisalze der Säuren Adipinsäure, Arachinsäure, Bernsteinsäure, Laurinsäure, Maleinsäure, Malonsäure, Myristinsäure, Ölsäure, Palmitinsäure, Stearinsäure und/oder Weinsäure umfassen sind daher nicht erfindungsgemäß.

Ebenso handelt es sich bei den erfindungsgemäßen Zubereitungen vorteilhaft um antitranspirant wirksame, schweißhemmende Zubereitungen allein aus der erfindungsgemäßen Kombination aus wasserlöslichem Erdalkalisalz, Carbonsäure und pH-Wert unter 5. Auch hierin unterscheiden sich die erfindungsgemäßen Zubereitungen von Zubereitungen des Standes der Technik, die einen anderen AT-wirkstoff enthalten.

Erfindungsgemäß bevorzugt sind antitranspirant wirksame kosmetische oder dermatologische Zubereitungen umfassend
- ein oder mehrere Erdalkalisalze ausgewählt aus der Gruppe der Magnesium- und Calciumhalogenide, insbesondere Magnesium- und Calciumchlorid, bevorzugt MgCl₂*6H₂O,
- ein oder mehrere Carbonsäuren ausgewählt aus der Gruppe Adipinsäure, Arachinsäure, Laurinsäure, Myristinsäure, Caprylsäure, Caprinsäure, Ölsäure, Plamitinsäure, Behensäure, Linolensäure, Stearinsäure und/oder Weinsäure, insbesondere Stearinsäure und Palmitinsäure,
- Wasser und/oder Ethanol
- Isosteareth-20 und
- ein oder mehrere Emulgatoren aus der Gruppe der linearen Emulgatoren, insbesondere Steareth-20 (HLB 15.3), Steareth-21 (HLB 15.5) und/oder Steareth-2 (HLB 4,9).
wobei der pH-Wert der Zubereitung vorteilhaft im Bereich von 3 bis 5, insbesondere im Bereich von 3,5 bis 4,5, bevorzugt im Bereich von 3,8 bis 4,2, gewählt wird.

Der Anteil an einem oder mehreren Erdalkalisalzen wird bevorzugt im Bereich von 3 bis 20 Gew.%, insbesondere im Bereich von 3 bis 10 Gew.% gewählt, bezogen auf die Gesamtmasse der Zubereitung.

Der Anteil an einer oder mehreren Carbonsäuren wird vorteilhaft im Bereich von 1 bis 5 Gew.%, insbesondere Im Bereich von 2 bis 4 gewählt, bezogen auf die Gesamtmasse der Zubereitung.

Der Anteil an einem oder mehreren linearen Emulgatoren wird vorteilhaft im Bereich von 2 bis 10 Gew.%, insbesondere 4 bis 8 Gew.%, gewählt.

Vorteilhaft werden neben den Erdalkalisalzen und Carbonsäuren keine weiteren antitranspirantwirksamen Stoffe, insbesondere keine Aluminiumsalze wie Aluminiumchlorohydrate, den erfindungsgemäßen Zubereitungen zugesetzt.

Der Zusatz an weiteren antitranspirant wirkenden, schweißhemmenden Stoffen liegt daher vorteilhaft unter 0,1 Gew.%, insbesondere bei 0 Gew.%, bezogen auf die Gesamtmasse der Zubereitung, um durch Einschleppungen oder Verunreinigungen auch diese als frei von zusätzlichen AT-wirkstoffen zu bezeichnen.

Erfindungsgemäße Zubereitungen sind bevorzugt O/W-Emulsionen, W/O-Emulsionen, PIT-Emulsionen oder Hydrodispersionen.

Die dargestellten Ergebnisse zeigten sich insbesondere vorteilhaft sowohl für Mikro- und Makroemulsionen.

Vorteilhaft umfassen die erfindungsgemäßen Zubereitungen ein oder mehrere Lipide.

Bevorzugt einzusetzende Lipide werden gewählt aus der Gruppe der Capric/Caprylic Triglycerid, Cocoglycerid, PPG-14 Butylether, PPG-9 Butylether, PPG-12 Butylether, PPG-14 Butylether, PPG-15 Butylether, PPG-16 Butylether, PPG-17 Butylether, PPG-18 Butylether, PPG-2 Butylether, PPG-20 Butylether, PPG-22 Butylether, PPG-24 Butylether, PPG-26 Butylether, PPG-30 Butylether, PPG-33 Butylether, PPG-40 Butylether, PPG-52 Butylether, PPG-53 Butylether, PPG-15 Stearylether, Coco-Caprylate/Caprate, Dicaprylyl Ether, Octyldodecanol, Paraffinum Liquidum, Isododecane, Isopropyl Palmitate, Persea Gratissima, Caprylyl Carbonate, Helianthus Annuus, Ethylhexyl Cocoate, C12-15 Alkyl Benzoate, Cyclomethicone und/oder Dimethicone, Phenyltrimethicon, Sonnenblumenöl, Rapsöl, Capric/Caprylic Triglycerid, Isopropyl Myristate, Isopropylpalmitat, Isopropyl Stearate, Cetearyl Ethylhexanoate, Hydrogenated Polydecene, Glycine Soja (Soybean) Oil, Olivenöl außerdem Guerbet-Alkohole wie beispielsweise Hexyldecanol, Octyldodecanol und 2- Ethylhexylalkohol, ferner auch Guerbetalkoholester, sowie Mischungen aus Guerbetalkoholen und Guerbetalkoholestem, wie z.B. Hexyldecanol und Hexyldecyllaurat.

Als Emulsion sind den erfindungsgemäßen Zubereitungen zusätzlich ein oder mehrere Emulgatoren aus der Gruppe der linearen Emulgatoren, wie beispielsweise Steareth-20 (HLB 15.3), Steareth-21 (HLB 15.5) und/oder Steareth-2 (HLB 4,9) zugesetzt.

Zur Stabilisierung der Emulsionen kann es förderlich sein, Strukturgeber oder Verdicker zuzusetzen, wobei die bevorzugte Viskosität für Roll-on Applikationen berücksichtigt bleiben sollte.

Beispiele bevorzugter Rheologiemodifizierer sind natürliche organische Polymere und ihre Derivate, Gummi arabicum, Karaya, Tragant, Johannisbrotkernmehl, Guar, Pektin, Agar agar, Carrageen, Alginate, Xanthan, Stärke und Stärkederivate, Cellulose und Cellulosederivate, wie Microcristalline Cellulose,Methylcellulose, Cellulose Gum, Hydroxyethylcellulose, Hydroxylpropylcellulose, Methylhydroxypropylcellulose, desweiteren anorganische Gelbildner, wie Silikate, z.B. Bentonite, Hectorite, kolloidale Kieselsäure; darüber hinaus synthetische Verdicker z.B. Poylvinylalkohol und ebenso ethoxylierte Verbindungen wie Poloxamer, PEG-150 Distearate, PEG-120 Methyl Glucose Dioleate, PEG-9 Dilaurate und Fettalkohole, wie Stearylalkolhol, Cetylalkohol und Cetearylalkohol.

Bevorzugt sind beispielsweise Hydroxyethylcellulosen, Hydroxypropylcellulose, Hydroxypropyl Methylcellulose, Ethylcellulose, Cellulose Gum, Xanthan-Gum, Guar Gum, Gellan Gum, Polyquaternium-37, Johannesbrotkernmehl, Hydroxyalkylstärkephosphate, und Carrageenan.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc.. Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

Zur Stabilisierung der pH-Wertes der Formulierungen können weitere Säuren und deren Puffersysteme mit geeigneten Alkalisierungsmitteln eingesetzt werden. Geeignete Säuren können Citronensäure, Milchsäure, Maleinsäure, Malonsäure, Bernsteinsäure, Hydroxybernsteinsäure (Äpfelsäure), Fumarsäure, Salicylsäure, Etidronsäure, Phosphorsäure, Salzsäure und Schwefelsäure sein.

Geeignete Alkalisierungsmittel zum Erstellen eines Puffersystems können beispielsweise Natriumhydroxid, Kaliumhydroxid, Ammoniak, Mono-, Di- und Trialkylamine sowie der Hydroxyalkylamine, Aminomethyl Propanol (2-Amino-2-methylpropan-1-ol), Ethanolamin (2-Aminoethanol), Triethanolamin (2,2',2"-Nitrilotriethanol) und Tetrahydroxypropyl ethylendiamin (1,1',1",1‴-Ethylendinitrilotetrapropan-2-ol) sein.

Um neben einer antitranspiranten Wirkung zeitgleich desodorierende Effekte zu ermöglichen werden den erfindungsgemäßen Zubereitungen vorteilhaft ein oder mehrere desodorierende Wirkstoffe zugesetzt.

Diese desodorierenden Wirkstoffe können bevorzugt gewählt sein aus kationischen Polymeren, insbesondere Polyquaternium-16, Polyquaternium-7, Polyquaternium-6, Polyquaternium-11 und Polyquaternium 37, Polyaminopropylbiguanid und epsilon-Polylysin.

Als desodorierende Wirkstoffe können ebenfalls vorteilhaft Octenidinhydrochlorid, Alexidindihydrochlorid, Benzylalkohol, Benzalkoniumchlorid, Cetyltrimethylamoniumchlorid, Silbercitrat, Triclosan, Ethylhexylglycerin, Triethylcitrat, 2-Butyloctansäure, Methylphenylbutanol, Phenoxyethanol, Zinkricinoleat und weitere Wirkstoffe, die die Anzahl an Bakterien auf der Haut reduzieren, eingesetzt werden.

Die erfindungsgemäßen Zubereitungen umfassen daher bevorzugt ferner ein oder mehrere Polyquaternium Polymere.

Erfindungsgemäß werden Polyquaternium Polymere aus der Gruppe der Polyquaternium-16 Polymere und Polyquaternium-6 Polymere ausgewählt. Diese sind in erfindungsgemäßen Zubereitungen in einem Anteil von 0,1 bis 10 Gew.%, insbesondere in einem Anteil von 0,15 bis 5 Gew.%, bezogen auf die Gesamtmasse der Zubereitung, enthalten.

Bevorzugt werden Polyquaternium-16 Polymere (3-Methyl-1-Vinylimidazolium Chlorid-1-Vinyl-2-Pyrrolidinon Chloride) ausgewählt. Erfindungsgemäß zeigen die eingesetzten Polyquaternium Polymere und insbesondere PQ-16 Polymere eine zusätzliche antimikrobielle Wirksamkeit.

In vorteilhaften Ausführungsformen der Erfindung können neben den eingesetzten Polyquaternium Polymeren, ein oder mehrere weitere desodorierend wirkende Stoffe enthalten sein.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Lipide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Viskosität, pH-Werte und AT-wirkung nicht beeinträchtigen oder ausgeschlossen sind.

Erstaunlicherweise zeigen die erfindungsgemäßen Zubereitungen auch in der Herstellung einige Vorteile. Insbesondere sind aufgrund der verringerten Viskosität Kosten und Aufwand für den Transport in den Produktionsleitungen und -gefäßen herabgesetzt.

Nachfolgende Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitung bezogen.

### Beispiele

| | 1 | 2 | 3 | 4 | 5 |
|---|---|---|---|---|---|
| Persea Gratissima Oil | 0,1 | 0,1 | 0,1 | 0,1 | 0,1 |
| Steareth-21 | 1,4 | 2 | 1,5 | 2 | 1 |
| Steareth-2 | 4,1 | 3 | 4 | 4 | 4 |
| Octyldodecanol | 0,1 | 3 | | | |
| PPG-15 Stearyl Ether | 3 | | | | |
| Capric/Caprylic Triglyceride | | | 3 | | |
| Isopropylpalmiate | | | | 3 | |
| PPG-14 Buthyl Ether | | | | | 3 |
| Phenoxyethanol | 0,3 | 0,3 | 0,3 | 0,3 | 0,3 |
| Isosteareth-20 | 1 | 0,5 | 1 | 1 | 1 |
| Palmitin acid+Stearic acid+Myristic acid arachidic acid+oleis acid | 2 | 2 | 2 | 3 | 2 |
| Magnesium Chlorid | 5 | 5 | 10 | 5 | 5 |
| Propylene Glycol | 3 | | 3 | 3 | |
| Octenidine HCl | 0,5 | | 0,5 | 0,5 | |
| Polyquaternium-16 | | 1 | | | |
| Polyquaternium-6 | | | | | 2 |
| Parfuem | 1 | 1 | 1 | 1 | 1 |
| Aqua | add 100 | add 100 | add 100 | add 100 | add 100 |
| Citric acid | pH=4 | pH=4 | pH=4 | pH=4 | pH=4 |

| | 6 | 7 | 8 | 9 |
|---|---|---|---|---|
| Persea Gratissima Oil | 0,1 | 0,1 | 0,1 | 0,1 |
| Steareth-21 | 2,5 | 2,5 | 3 | 2,5 |
| Steareth-2 | 3,5 | 3,5 | 4 | 3,5 |
| Capric/Caprylic Triglyceride | 3 | | | |
| C12-15 Alkyl Benzoate | | 3 | | 3 |
| Dicaprylyl Ether | | | 3 | |
| Phenoxyethanol | 0,3 | 0,3 | 0,3 | 0,3 |
| Isosteareth-20 | 0,5 | 0,5 | 1 | 2 |
| Palmitin acid+Stearic acid+Myristic acid arachidic acid+oleis acid | 2 | 2 | 3,5 | 2 |
| Magnesium Chlorid | 7,5 | 5 | 5 | 5 |
| Propylene Glycol | | 3 | | |
| Octenidine HCl | | 0,5 | | |
| Polyquaternium-16 | 1 | | 1 | |
| Polylysin | | | | 0,5 |
| Parfuem | 1 | 1 | 1 | 1 |
| Aqua | add 100 | add 100 | add 100 | add 100 |
| Citric acid | pH=4 | pH=4 | pH=4 | pH=4 |

## Patentansprüche

1. Kosmetische oder dermatologische Zubereitung umfassend
- Wasser und/oder Alkohol,
- ein oder mehrere wasserlösliche Erdalkalimetallsalze,
- ein oder mehrere Carbonsäuren und
- ein oder mehrere Emulgatoren aus der Gruppe der Gemische aus Polyethylenglycolethern mit verzweigten Stearylalkoholen
**dadurch gekennzeichnet, dass** der pH-Wert der Zubereitung im Bereich bis 5 gewählt wird.

2. Kosmetische oder dermatologische Zubereitung nach Anspruch 1 umfassend
- Wasser und/oder Alkohol,
- ein oder mehrere wasserlösliche Erdalkalimetallsalze,
- ein oder mehrere Carbonsäuren und
- Isosteareth-20.

3. Zubereitung nach Anspruch 1 oder 2 **dadurch gekennzeichnet, dass** zusätzlich ein oder mehrere Emulgatoren aus der Gruppe der linearen Emulgatoren, insbesondere Steareth-20, Steareth-21 und/oder Steareth-2 zugesetzt werden.

4. Zubereitung nach Anspruch 1, 2 oder 3 **dadurch gekennzeichnet, dass** der pH-Wert der Zubereitung im Bereich 3,5 bis 4,5, insbesondere im Bereich von 3,8 bis 4,2, gewählt wird.

5. Zubereitung nach einem der vorstehenden Ansprüche **dadurch gekennzeichnet, dass** der Anteil an Emulgatoren der Gemische aus Polyethylenglycolethern mit verzweigten Stearylalkoholen, insbesondere Isosteareth-20, im Bereich von 0,5 bis 2 Gew.%, bezogen auf die Gesamtmasse der Zubereitung, gewählt wird.

6. Zubereitung nach einem der vorstehenden Ansprüche **dadurch gekennzeichnet, dass** als Erdalkalimetallsalze Magnesium-, Calcium- und/oder Strontiumhalogenide und/oder Magnesiumphosphat oder - lactat gewählt werden.

7. Zubereitung nach Anspruch 6 **dadurch gekennzeichnet, dass** als Erdalkalimetallsalze Calciumchlorid und/oder Magnesiumchlorid und/oder Hydrate des Magnesiumchlorid, insbesondere MgCl₂*6H₂O, gewählt werden.

8. Zubereitung nach einem der vorstehenden Ansprüche **dadurch gekennzeichnet, dass** als Carbonsäure Adipinsäure, Arachinsäure, Bernsteinsäure, Laurinsäure, Maleinsäure, Malonsäure, Myristinsäure, Caprylsäure, Caprinsäure, Ölsäure, Palmitinsäure, Behensäure, Linolensäure, Stearinsäure und/oder Weinsäure gewählt werden.

9. Zubereitung nach einem der vorstehenden Ansprüche **dadurch gekennzeichnet, dass** als Carbonsäure Palmitinsäure, Stearinsäure, Myristinsäure, Ölsäure und/oder Arachinsäure, insbesondere Stearinsäure und/oder Palmitinsäure, gewählt werden.

10. Zubereitung nach einem der vorstehenden Ansprüche **dadurch gekennzeichnet, dass** der Anteil an einem oder mehreren Erdalkalimetallsalzen im Bereich von 5 bis 20 Gew.%, insbesondere im Bereich von 7 bis 10 Gew.% gewählt wird, bezogen auf die Gesamtmasse der Zubereitung.

11. Zubereitung nach einem der vorstehenden Ansprüche **dadurch gekennzeichnet, dass** der Anteil an einer oder mehreren Carbonsäuren im Bereich von 1 bis 5 Gew.%, insbesondere im Bereich von 1,5 bis 4 Gew.% gewählt wird, bezogen auf die Gesamtmasse der Zubereitung.

12. Zubereitung nach einem der vorstehenden Ansprüche **dadurch gekennzeichnet, dass** sie in Form einer Mikro- oder Makroemulsionen vorliegen.

13. Verwendung einer Zubereitung nach einem der Ansprüche 1 bis 12 in Spray- oder Roll-on Applikatoren.

14. Verwendung von ein oder mehrere Emulgatoren aus der Gruppe der Gemische aus Polyethylenglycolethern mit verzweigten Stearylalkoholen, insbesondere Isosteareth-20, in kosmetischen oder dermatologischen Zubereitungen umfassend Wasser und/oder Alkohol, ein oder mehrere wasserlösliche Erdalkalimetallsalze und ein oder mehrere Carbonsäuren, zur Verringerung der Viskosität der Zubereitung.

## Claims

1. Cosmetic or dermatological preparation comprising
- water and/or alcohol,
- one or more water-soluble alkaline earth metal salts,
- one or more carboxylic acids and
- one or more emulsifiers from the group of mixtures of polyethylene glycol ethers with branched stearyl alcohols,
**characterized in that** the pH of the preparation is selected in the range up to 5.

2. Cosmetic or dermatological preparation according to Claim 1, comprising
- water and/or alcohol,
- one or more water-soluble alkaline earth metal salts,
- one or more carboxylic acids and
- Isosteareth-20.

3. Preparation according to Claim 1 or 2, **characterized in that** one or more emulsifiers from the group of linear emulsifiers, in particular Steareth-20, Steareth-21 and/or Steareth-2, are additionally added.

4. Preparation according to Claim 1, 2 or 3, **characterized in that** the pH of the preparation is selected in the range 3.5 to 4.5, in particular in the range from 3.8 to 4.2.

5. Preparation according to any of the preceding claims, **characterized in that** the proportion of emulsifiers in the mixtures of polyethylene glycol ethers with branched stearyl alcohols, in particular Isosteareth-20, is selected in the range from 0.5% to 2% by weight, based on the total mass of the preparation.

6. Preparation according to any of the preceding claims, **characterized in that** alkaline earth metal salts selected are magnesium halides, calcium halides and/or strontium halides and/or magnesium phosphate or magnesium lactate.

7. Preparation according to Claim 6, **characterized in that** alkaline earth metal salts selected are calcium chloride and/or magnesium chloride and/or hydrates of magnesium chloride, in particular MgCl₂*6H₂O.

8. Preparation according to any of the preceding claims, **characterized in that** the carboxylic acid selected is adipic acid, arachidic acid, succinic acid, lauric acid, maleic acid, malonic acid, myristic acid, caprylic acid, capric acid, oleic acid, palmitic acid, behenic acid, linolenic acid, stearic acid and/or tartaric acid.

9. Preparation according to any of the preceding claims, **characterized in that** the carboxylic acid selected is palmitic acid, stearic acid, myristic acid, oleic acid and/or arachidic acid, in particular stearic acid and/or palmitic acid.

10. Preparation according to any of the preceding claims, **characterized in that** the proportion of one or more alkaline earth metal salts is selected in the range from 5% to 20% by weight, in particular in the range from 7% to 10% by weight, based on the total mass of the preparation.

11. Preparation according to any of the preceding claims, **characterized in that** the proportion of one or more carboxylic acids is selected in the range from 1% to 5% by weight, in particular in the range from 1.5% to 4% by weight, based on the total mass of the preparation.

12. Preparation according to any of the preceding claims, **characterized in that** it is in the form of a microemulsion or macroemulsion.

13. Use of a preparation according to any of Claims 1 to 12 in spray or roll-on applicators.

14. Use of one or more emulsifiers from the group of mixtures of polyethylene glycol ethers with branched stearyl alcohols, in particular Isosteareth-20, in cosmetic or dermatological preparations comprising water and/or alcohol, one or more water-soluble alkaline earth metal salts and one or more carboxylic acids, to reduce the viscosity of the preparation.

## Revendications

1. Préparation cosmétique ou dermatologique comprenant
- de l'eau et/ou un alcool,
- un ou plusieurs sels hydrosolubles de métaux alcalino-terreux,
- un ou plusieurs acides carboxyliques et
- un ou plusieurs émulsifiants choisis dans le groupe des mélanges d'éthers de polyéthylèneglycol avec des alcools stéaryliques ramifiés
**caractérisée en ce que** le pH de la préparation est choisi dans l'intervalle allant jusqu'à 5.

2. Préparation cosmétique ou dermatologique selon la revendication 1, comprenant
- de l'eau et/ou un alcool,
- un ou plusieurs sels hydrosolubles de métaux alcalino-terreux,
- un ou plusieurs acides carboxyliques et
- de l'Isosteareth-20

3. Préparation selon la revendication 1 ou 2, **caractérisée en ce qu'**en plus sont ajoutés un ou plusieurs émulsifiants choisis dans le groupe des émulsifiants linéaires, en particulier du Steareth-20, Steareth-21 et/ou Steareth-2.

4. Préparation selon la revendication 1, 2 ou 3, **caractérisée en ce que** le pH de la préparation est choisi dans l'intervalle de 3,5 à 4,5, en particulier dans l'intervalle de 3,8 à 4,2.

5. Préparation selon l'une quelconque des revendications précédentes, **caractérisée en ce que** la proportion d'émulsifiants des mélanges d'éthers de polyéthylèneglycol avec des alcools stéaryliques ramifiés, en particulier d'Isosteareth-20, est choisie dans la plage de 0,5 à 2 % en poids, par rapport à la masse totale de la préparation.

6. Préparation selon l'une quelconque des revendications précédentes, **caractérisée en ce que** des halogénures de magnésium, calcium et/ou strontium et/ou le phosphate ou lactate de magnésium sont choisis en tant que sels de métaux alcalino-terreux .

7. Préparation selon la revendication 6, **caractérisée en ce que** le chlorure de calcium et/ou le chlorure de magnésium et/ou des hydrates du chlorure de magnésium, en particulier MgCl₂*6H₂O, sont choisis en tant que sels de métaux alcalino-terreux.

8. Préparation selon l'une quelconque des revendications précédentes, **caractérisée en ce que** l'acide adipique, l'acide arachidique, l'acide succinique, l'acide laurique, l'acide maléique, l'acide malonique, l'acide myristique, l'acide caprylique, l'acide caprique, l'acide oléique, l'acide palmitique, l'acide béhénique, l'acide linolénique, l'acide stéarique et/ou l'acide d-tartrique sont choisis en tant qu'acide carboxylique.

9. Préparation selon l'une des revendications précédentes, **caractérisée en ce que** l'acide palmitique, l'acide stéarique, l'acide myristique, l'acide oléique et/ou l'acide arachidique, en particulier l'acide stéarique et/ou l'acide palmitique, sont choisis en tant qu'acide carboxylique.

10. Préparation selon l'une quelconque des revendications précédentes, **caractérisée en ce que** la proportion d'un ou de plusieurs sels de métaux alcalino-terreux est choisie dans la plage de 5 à 20 % en poids, en particulier dans la plage de 7 à 10 % en poids, par rapport à la masse totale de la préparation.

11. Préparation selon l'une quelconque des revendications précédentes, **caractérisée en ce que** la proportion d'un ou de plusieurs sels de métaux alcalino-terreux est choisie dans la plage de 1 à 5 % en poids, en particulier dans la plage de 1,5 à 4 % en poids, par rapport à la masse totale de la préparation.

12. Préparation selon l'une quelconque des revendications précédentes, **caractérisée en ce qu'**elle se présente sous forme d'une micro- ou macro-émulsion.

13. Utilisation d'une préparation selon l'une quelconque des revendications 1 à 12 dans des applicateurs par pulvérisation ou à bille.

14. Utilisation d'un ou de plusieurs émulsifiants du groupe des mélanges d'éthers de polyéthylèneglycol avec des alcools stéaryliques, en particulier d'Isosteareth-20, dans des préparations cosmétiques ou dermatologiques comprenant de l'eau et/ou un alcool, un ou plusieurs sels hydrosolubles de métaux alcalino-terreux et un ou plusieurs acides carboxyliques, pour la diminution de la la viscosité de la préparation.
